# EUROPEAN PATENT APPLICATION

(11) **EP 4 183 438 A1**
(43) Date of publication of application: **24.05.2023**
(21) Application number: 20945024.6
(22) Date of filing: 16.07.2020
(51) Int. Cl.: A61M 25/00, A61M 25/10

(54) **CATHETER**

(71) Applicant: Asahi Intecc Co., Ltd., Seto-shi, Aichi 489-0071 (JP)
(72) Inventor: KATSURADA, Takeharu, Seto-shi, Aichi 489-0071 (JP)
(74) Representative: Prinz & Partner mbB
(86) International application number: PCT/JP2020/027625
(87) International publication number: WO 2022/014000

(57) **Abstract**

An object of the disclosure is to provide a catheter capable of preventing directionality from being exhibited when bent and having improved operability.

A catheter 1 includes a hollow shaft 51 and an outer shaft 41 arranged at an outer periphery of the hollow shaft 51. The hollow shaft 51 has the slit part 52 in which a plurality of slits 52b extending spirally is formed. In the slit part 52, the proximal ends of the slits 52b are each formed at a same axial position and at equal intervals in the circumferential direction.

## Description

### [Technical Field]

This disclosure relates to a catheter.

### [Background Art]

A catheter or the like is used to treat a site in a body cavity such as a blood vessel. As one of such catheters, for example, a catheter including a metal hypotube, a resin outer tube provided on a position closer to a distal end side (balloon side) than the hypotube, and a core wire provided across the hypotube and the outer tube is known (see, for example, Patent Literature 1).

As another such catheter, for example, a catheter including a metal hypotube, a spiral notch on a distal end side (balloon side) of the hypotube, and a resin outer tube covering the hypotube is known (see, for example, Patent Literature 2).

### [Citation List]

### [Patent Literature]

[Patent Literature 1] Japanese Patent Application Publication No. 2013-111264
[Patent Literature 2] Japanese Patent Application Publication No. 2002-253678

### [Summary of Invention]

### [Technical Problem]

In the above catheter, the core wire is provided as a rigid reinforcing material, and is fixed to a portion of an inner peripheral surface of the hypotube in a circumferential direction. Hence, the directionality is exhibited when the hypotube and the outer tube are bent, resulting in poor operability.

An object of the disclosure is to provide a catheter capable of preventing directionality from being exhibited when bent and having improved operability.

### [Solution to Problem]

Some aspects of this disclosure include:
(1) a catheter, including a hollow shaft and an outer shaft arranged at an outer periphery of the hollow shaft, wherein the hollow shaft has a slit part, in which a plurality of slits extending spirally is formed, and in the slit part, proximal ends of the slits are each formed at a same axial position and at equal intervals in the circumferential direction;
(2) the catheter according to (1) above, wherein the hollow shaft in the slit part is configured to have a radial thickness that gradually decreases from the proximal end to the distal end;
(3) the catheter according to (1) or (2) above, wherein the slit part is configured of a hollow rope body and is connected to a tubular body arranged at the proximal end side of the slit part;
(4) the catheter according to any one of (1) to (3) above, wherein each slit is configured to have a width in such a manner that the width at the distal end is wider than the width at the proximal end; and
(5) the catheter according to any one of (1) to (4) above, further having a balloon, wherein the hollow shaft communicates with the balloon.

### [Advantageous Effects of Invention]

The disclosure can provide a catheter capable of preventing the directionality from being exhibited when bent, and having improved operability.

### [Brief Description of Drawings]

FIG. 1 is a schematic longitudinal sectional view of a catheter according to an embodiment of the disclosure.
FIG. 2 is an enlarged sectional view of a portion in the vicinity of a slit part.
FIG. 3 is an end view of the proximal end side of the slit part.

### [Description of Embodiments]

The catheter includes a hollow shaft and an outer shaft arranged at an outer periphery of the hollow shaft, wherein the hollow shaft has a slit part, in which a plurality of slits extending spirally is formed, and proximal ends of the slits are each formed at a same axial position and at equal intervals in the circumferential direction.

Hereinafter, the catheter according to the embodiment is described with reference to the enclosed drawings. However, the disclosure is not limited to the embodiment described in the drawings. In the following embodiment, the catheter being a balloon catheter will be illustrated and described. Moreover, the size of the catheter in drawings is a size illustrated to facilitate understanding of the embodiment, and does not correspond to the actual size.

Further, in the Description, a "distal end side (distal side)" indicates a side on which a distal tip is located in the longitudinal direction of the catheter. A "proximal end side (proximal side)" indicates a side opposite to the distal end side in the longitudinal direction. A "distal end" indicates an end part on the distal end side of each member forming the catheter. A "proximal end" indicates an end part on the proximal end side of each member forming the catheter.

Hereinafter, a catheter 1 according to the embodiment of the disclosure is described with reference to the enclosed drawings.

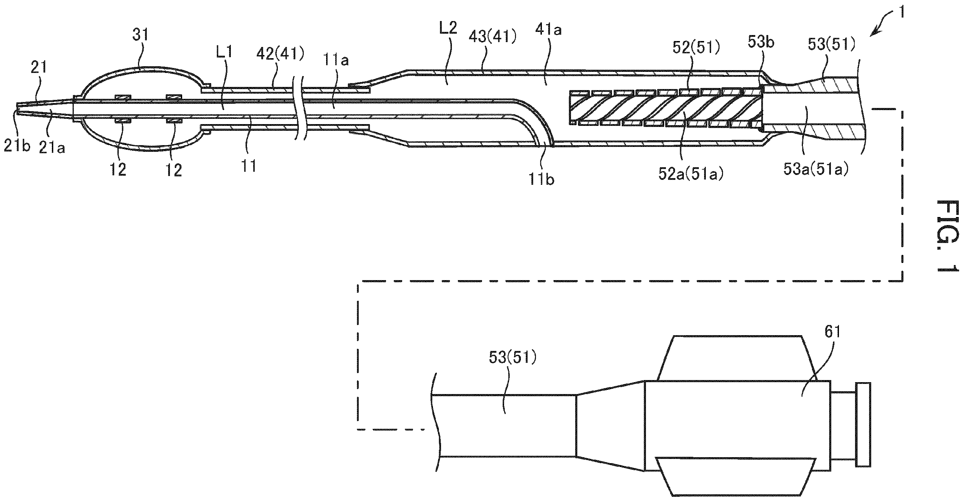

FIG. 1 is a schematic longitudinal sectional view of the catheter 1 according to the embodiment.

As illustrated in FIG. 1, the catheter 1 is schematically configured of an inner shaft 11, a distal tip 21, a balloon 31, an outer shaft 41, a hollow shaft 51, and a connector 61.

The inner shaft 11 is a cylindrical (hollow-shaped) shaft. The inner shaft 11 has an inner cavity 11a penetrating along the longitudinal direction. The distal end of the inner shaft 11 can be connected to, for example, a distal tip 21 described later. A proximal end portion of the inner shaft 11 is connected to, for example, a second outer shaft portion 43 of the outer shaft 41 described later, and can be arranged so that an opening 11b at the proximal end of the inner cavity 11a faces the outside. Two annular markers 12 are attached to an outer periphery of a portion covered by the balloon 31 described later of the inner shaft 11. The markers 12 are made of a radiopaque material.

The distal tip 21 is a cylindrical (hollow-shaped) member connected to the distal end of the inner shaft 11. The distal tip 21 has an inner cavity 21a penetrating along the longitudinal direction and is formed in such a manner that the distal end portion is substantially sharp-shaped toward the distal end side. The catheter 1 includes the distal tip 21, so as to be able to lower the resistance to be applied when the catheter advances in the body cavity, allowing smooth advancement of the catheter 1, for example.

Here, the above inner cavity 11a and the inner cavity 21a communicate with each other, so as to form a lumen L1. For example, a medical device or the like (not shown) such as a guide wire or a treatment device is inserted into the lumen L1.

The balloon 31 is an expandable and contractable member arranged so as to cover at least a part of the outer periphery of the inner shaft 11. For example, the distal end of the balloon 31 is joined to the distal end of the inner shaft 11 and/or the proximal end of the distal tip 21, and the proximal end of the balloon 31 is joined to the distal end of the outer shaft 41 described later. The balloon 31 is inflated by injecting a liquid into it, and can, for example, enlarge the vascular inner wall or expand a stent.

The outer shaft 41 is a cylindrical (hollow-shaped) shaft arranged so as to cover the proximal end side portion of the inner shaft 11 and a slit part 52 of the hollow shaft 51 described later. Inside the outer shaft 41, an inner cavity 41a extending along the longitudinal direction is formed between the outer shaft 41, and the inner shaft 11 and the slit part 52. The distal end of the outer shaft 41 is joined to the proximal end of the balloon 31. The outer shaft 41 is configured in the order of a first outer shaft portion 42 and the second outer shaft portion 43 from the distal end side. The first outer shaft portion 42 and the second outer shaft portion 43 may be integrated or may be joined separately.

The distal end of the first outer shaft portion 42 is joined to the proximal end of the balloon 31. Inside the first outer shaft portion 42, the inner shaft 11 is inserted.

The second outer shaft portion 43 is located on the proximal end side of the first outer shaft portion 42. The distal end of the second outer shaft portion 43 is joined to the proximal end of the first outer shaft portion 42. The proximal end of the second outer shaft portion 43 is joined to the distal end of a shaft main body portion 53 of the hollow shaft 51 described later. Inside the second outer shaft portion 43, the proximal end portion of the inner shaft 11 and the slit part 52 are inserted.

Materials for forming the inner shaft 11, the distal tip 21, the balloon 31 and the outer shaft 41 are preferably anti-thrombotic, flexible, and biocompatible because they are inserted into body cavities. Examples of the above materials that can be employed herein include resin materials or the like such as polyamide, polyamide elastomer, polyolefin, polyester, polyester elastomer, polyurethane, polyurethane elastomer, silicone, and fluorocarbon polymers.

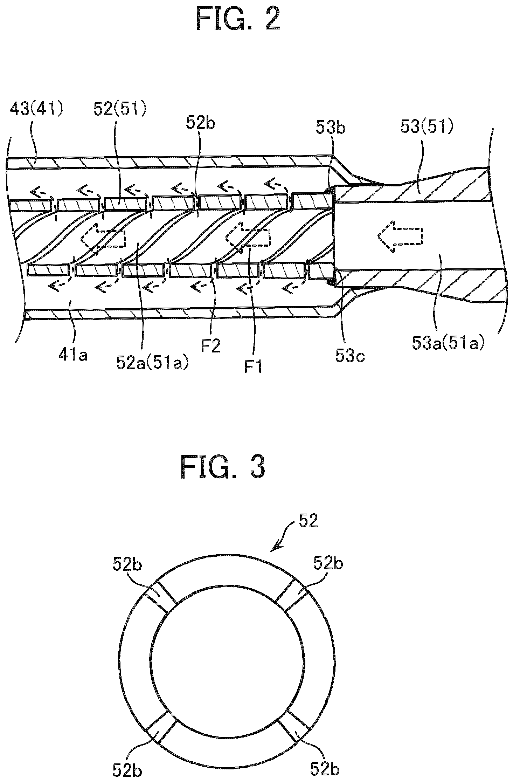

FIG. 2 is an enlarged sectional view of a portion in the vicinity of the slit part 52.

FIG. 3 is an end view of the proximal end side of the slit part 52.

The hollow shaft 51 is a cylindrical (hollow-shaped) shaft, as shown in FIGS. 1 and 2. The hollow shaft 51 has an inner cavity 51a penetrating along the longitudinal direction. The hollow shaft 51 has the slit part 52 and the shaft main body portion 53. The slit part 52 functions as a reinforcing body for reinforcing the rigidity of the second outer shaft portion 43.

The slit part 52 is located in the second outer shaft portion 43. The slit part 52 is configured of a hollow rope body formed by twisting a plurality of (four in this embodiment) flat-shaped wires together, and has an inner cavity 52a penetrating along the longitudinal direction. The hollow shaft 51 in the slit part 52 is configured to have a radial thickness that gradually decreases from the proximal end to the distal end. Specifically, each wire is configured to have a thickness that gradually decreases from the proximal end to the distal end. The slit part 52 has a tapered shape such that it tapers from the proximal end side to the distal end side. The slit part 52 is configured to have a substantially fixed inner diameter in the longitudinal direction. Each wire is configured to have a substantially fixed width from the proximal end to the distal end.

A plurality of slits 52b extending spirally is formed in the slit part 52. Specifically, the slit part 52 is formed of a plurality of wires in such a manner that the wires are twisted together so as to have a gap between adjacent wires. The width of each slit 52b is formed in such a manner that the width at the distal end is wider than that at the proximal end. As shown in FIG. 3, which is an end view of the proximal end side of the slit part 52, in the slit part 52, the proximal ends of the slits 52b are each formed at a same axial position and at equal intervals in the circumferential direction. The inner cavity 52a of the slit part 52 communicates with the inner cavity 41a of the outer shaft 41 via an opening at the distal end of the slit part 52 and the slits 52b.

The shaft main body portion 53 is located on the proximal end side of the slit part 52. The shaft main body portion 53 has an inner cavity 53a penetrating along the longitudinal direction. The above inner cavity 52a and inner cavity 53a communicate with each other, and the inner cavity 52a and the inner cavity 53a form the inner cavity 51a of the hollow shaft 51. The inner cavity 41a of the outer shaft 41 and the inner cavity 51a of the hollow shaft 51 communicate with each other, so as to form an expansion lumen L2. The inner cavity 51a of the hollow shaft 51 communicates with the balloon 31 via the inner cavity 41a of the outer shaft 41. The distal end of the shaft main body portion 53 is joined to the proximal end of the slit part 52, for example, by laser welding or brazing (joint part 53b). The inner diameter of the shaft main body portion 53 is configured to be larger than that of the slit part 52. This results in a step difference 53c formed at the connection between the slit part 52 and the shaft main body portion 53. The shaft main body portion 53 corresponds to a tubular body.

Materials for forming the hollow shaft 51 are not particularly limited, and a superelastic alloy such as stainless steel (SUS304) or a Ni-Ti alloy can be used. The slit part 52 and the shaft main body portion 53 may be made of the same material or different materials.

The connector 61 is a member for the operator to hold the catheter 1, and is connected to the proximal end of the shaft main body portion 53. An indeflator (not shown) is attached to the connector 61. A liquid such as a contrast medium or a physiological saline is supplied from the indeflator (not shown) to the inner cavity 51a of the hollow shaft 51 via the connector 61.

Next, a use form of the catheter 1 is described. Here, the catheter 1 is a balloon catheter, and a procedure for expanding a constricted part (treatment site) existing in the coronary artery of the heart using the catheter 1 is described.

First, prior to inserting the catheter 1 (hereinafter, also referred to as "balloon catheter 1"), a guide wire (not shown) is inserted into a blood vessel and pushed along the blood vessel to the treatment site. Next, using the balloon catheter 1 in which the balloon 31 has a reduced diameter, the proximal end of the guide wire is inserted into an opening 21b of the lumen L1 of the balloon catheter 1 and then the balloon catheter 1 is inserted from the distal end thereof into the blood vessel.

Next, the balloon catheter 1 is advanced along the guide wire to the treatment site. In a state where the balloon 31 reaches the inside of a constricted part, a liquid such as a contrast medium or a physiological saline is supplied from the indeflator (not shown) to the expansion lumen L2 via the connector 61. At this time, as shown in FIG. 2, the liquid flows not only along the main path as shown as flow F1, along which the liquid flows in the inner cavity 52a of the slit part 52, but also partially along a sub path as shown as flow F2, along which the liquid flows to the inner cavity 41a between the slit part 52 and the second outer shaft portion 43 via the slits 52b. This can shorten the indeflation time. Then, the liquid supplied to the expansion lumen L2 flows into the balloon 31 to inflate the balloon 31. At this time, the outer peripheral surface of the inflated balloon 31 enlarges the constricted part while contacting the inner wall of the constricted part, thereby expanding the constricted part.

After the expansion of the constricted part is completed, the expansion solution is discharged from the balloon 31 via the expansion lumen L2 to reduce the diameter. Next, after the diameter of the balloon 31 is reduced, the balloon catheter 1 is retracted and removed from the body to complete the use of the balloon catheter 1.

As described above, in the catheter 1 of the embodiment, a plurality of slits 52b extending spirally is formed in the slit part 52 of the hollow shaft 51, and in the slit part 52, the proximal ends of the slits 52b are each formed at same axial position and at equal intervals in the circumferential direction. This can prevent directionality from being exhibited when the slit part 52 is bent, and by extension, this can prevent directionality from being exhibited when the outer shaft 41 and the hollow shaft 51 are bent. Therefore, the operability of the catheter 1 can be improved.

The hollow shaft 51 in the slit part 52 is configured to have a radial thickness that gradually decreases from the proximal end to the distal end. This can increase the flexibility from the proximal end side to the distal end side of the slit part 52 while reinforcing the rigidity of the second outer shaft portion 43. The configuration is made, so that when the radial thickness of the slit part 52 is gradually reduced toward the distal end, the outer diameter of the slit part 52 gradually decreases, and the intervals between the outer periphery of the slit part 52 and the inner periphery of the second outer shaft portion 43 are regular. This enables the outer shaft 41 to have the diameter decreasing toward the distal end.

The slit part 52 is configured of a hollow rope body and is connected to the shaft main body portion 53 arranged at the proximal end side of the slit part 52. Therefore, the slit part 52 can be easily provided.

The width of each slit 52b is formed in such a manner that the width at the distal end is wider than that at the proximal end. This can increase the flexibility from the proximal end side to the distal end side of the slit part 52 while reinforcing the rigidity of the second outer shaft portion 43. Since a liquid such as a contrast medium easily passes through the slits 52b on the distal end side of the slit part 52, it is possible to prevent a large pressure from being applied to the thin distal end portion of the slit part 52 by the liquid such as the contrast medium.

Note that the disclosure is not limited to the configuration of the above embodiment, but is defined by the terms of the claims and are intended to include any modifications within the scope and meaning equivalent to the terms of the claims. For example, a part of the configurations of the above embodiment may be deleted or replaced by another configuration, or another configuration may be added to the configuration of the above embodiment.

In the above embodiment, in the hollow shaft 51, the slit part 52 and the shaft main body portion 53 are configured as separate bodies, but they may be configured integrally. In this case, each slit 52b of the slit part 52 may be formed in a cylindrical body by laser processing or the like. The slit part 52, which is a hollow rope body, is configured of four wires, but the number of wires may be any number as long as it is two or more. The inner diameter of the slit part 52 is a substantially fixed inner diameter in the longitudinal direction, but may gradually decrease in the longitudinal direction. Each wire of the slit part 52 has a substantially fixed width from the proximal end to the distal end, but may be configured to taper. The catheter 1 is a balloon catheter as described above, but it may be another catheter.

### [Reference Signs List]

1 catheter
41 outer shaft
42 first outer shaft portion
43 second outer shaft portion
51 hollow shaft
52 slit part
52b slit

## Claims

1. A catheter, comprising:
a hollow shaft; and
an outer shaft arranged at an outer periphery of the hollow shaft,
wherein the hollow shaft has a slit part, in which a plurality of slits extending spirally is formed, and in the slit part, proximal ends of the slits are each formed at a same axial position and at equal intervals in the circumferential direction.

2. The catheter according to claim 1, wherein the hollow shaft in the slit part is configured to have a radial thickness that gradually decreases from proximal end to distal end.

3. The catheter according to claim 1 or 2, wherein the slit part is configured of a hollow rope body and is connected to a tubular body arranged at a proximal end side of the slit part.

4. The catheter according to any one of claims 1 to 3, wherein each slit is configured to have a width in such a manner that the width at a distal end is wider than the width at a proximal end.

5. The catheter according to claims 1 to 4, further having a balloon, wherein the hollow shaft communicates with the balloon.
